# EUROPÄISCHE PATENTSCHRIFT

(11) **EP 2 750 793 B1**
(45) Veröffentlichungstag und Bekanntmachung des Hinweises auf die Patenterteilung: **19.09.2018**
(21) Anmeldenummer: 12762553.1
(22) Anmeldetag: 29.08.2012
(51) Int. Cl.: B01J 23/62, B01J 23/64, B01J 23/648, B01J 35/00, B01J 35/02, B01J 35/10, C07C 209/36

(54) **KATALYSATOR UND VERFAHREN ZUR HERSTELLUNG VON AROMATISCHEN AMINEN IN DER GASPHASE**
CATALYST AND METHOD FOR PRODUCING AROMATIC AMINES IN THE GASEOUS PHASE
CATALYSEUR ET PROCÉDÉ DE PRÉPARATION D'AMINES AROMATIQUES EN PHASE GAZEUSE

(30) Priorität: 31.08.2011 DE 102011081897
(43) Veröffentlichungstag der Anmeldung: 09.07.2014
(73) Patentinhaber: Covestro Deutschland AG, 51373 Leverkusen (DE)
(72) Erfinder: MERKEL, Michael, 40223 Düsseldorf (DE); WILKE, Karl-Heinz, 47447 Moers (DE); LEHNER, Peter, 45481 Mülheim an der Ruhr (DE)
(74) Vertreter: Levpat
(86) Internationale Anmeldenummer: PCT/EP2012/066749
(87) Internationale Veröffentlichungsnummer: WO 2013/030221

(56) Entgegenhaltungen:
- EP-A1- 0 371 331
- EP-A2- 0 507 118
- DE-A1- 2 849 002

## Beschreibung

Die vorliegende Erfindung betrifft einen Katalysator gemäß Patentanspruch 1. Ein weiterer Gegenstand der vorliegenden Erfindung ist der Einsatz eines solchen Katalysators in der katalytischen Gasphasenhydrierung von Nitroaromaten.

Die Hydrierung von Nitrobenzol zu den entsprechenden aromatischen Aminen in der Gasphase an ortsfest angebrachten Palladium-Katalysatoren auf keramischen Trägern ist bekannt. So wird in DE 28 49 002 A1 ein Verfahren zur Reduktion von Nitroverbindungen in Gegenwart von Palladiumhaltigen Drei-Komponenten-Trägerkatalysatoren in gekühlten Rohrreaktoren beschrieben. Der Katalysator enthält in bevorzugten Ausführungsformen 1 bis 20 g Palladium, 1 bis 20 g Vanadium und 1 bis 20 g Blei pro Liter α-Al₂O₃. Ähnliche Katalysatoren, allerdings zusätzlich dotiert mit Mo, Re oder W, wurden auch in DE 197 15 746 A1 beschrieben. EP 1 882 681 A1 offenbart, dass es vorteilhaft ist, solche Drei-Komponenten-Trägerkatalysatoren zusätzlich mit einer Schwefel- oder Phosphor-haltigen, bevorzugt Phosphor-haltigen, Verbindung zu dotieren. Dabei werden Gehalte von 0,1 bis 2 Massen-%, bevorzugt von 0,1 bis 1 Massen-% Schwefel oder Phosphor offenbart. Als Beispiele für Phosphorhaltige Verbindungen werden die Sauerstoffsäuren des Phosphors oder deren Alkalisalze wie z. B. Natriumdihydrogenphosphat, Natrium- oder Kaliumphosphat oder Natriumhypophosphit genannt. Dem Gegenion Kalium wird dabei keine besondere Bedeutung beigemessen.

Nachteilig bei den in den erwähnten Patentveröffentlichungen beschriebenen Gasphasenhydrierungen ist die Bildung von phenolischen Nebenprodukten. So wird zum Beispiel für das einfachste aromatische Amin, das Anilin, ein erheblicher Aufwand betrieben, um das Nebenprodukt Phenol, dessen Siedetemperatur sich mit 182 °C nur um 2 K von der des Anilins unterscheidet, abzutrennen (siehe z. B. EP 1 670 747 B1**,** EP 2 028 176 A1**,** JP 2007 217405 (A**),** EP 1 845 079 A1**,** EP 1 845 080 A1**).** Der Haupteil des weltweit produzierten Anilins wird für die Herstellung von Methylendiphenyldiamin (MDA) zur Herstellung von Methylendiphenyldiisocyanat (MDI) verwendet. Wird das Phenol nicht zuvor abgetrennt, kontaminiert es im MDA-Prozess das Abwasser und muss dann in einer geeigneten Abwasserbehandlung ebenfalls aufwändig (z. B. Aktivkohlebehandlung, Ozonolyse, etc.) vernichtet werden.

Es bestand daher ein Bedarf an einem Verfahren zur Herstellung von aromatischen Aminen in der Gasphase durch katalytische Hydrierung der korrespondierenden Nitroverbindungen, bei dem die Bildung von phenolischen Verbindungen durch die Verwendung eines speziellen Katalysators weitgehend unterdrückt wird, ohne dass sich dadurch andere Faktoren wie die Betriebszeit des Katalysators verschlechtern, so dass der Aufwand für die Abtrennung bzw. Vernichtung dieser phenolischen Verbindungen reduziert wird.

Diesem Bedarf Rechnung tragend ist ein Gegenstand der vorliegenden Erfindung ein Katalysator umfassend α-Aluminumoxid als keramischen Träger mit einer BET-Oberfläche von weniger als 10 m²/g, und, bezogen jeweils auf die Summe aller Vertreter einer Komponente (a)-(c),
(a) 8,0 g bis 100 g, bevorzugt 8,0 g bis 50 g, wenigstens eines Metalls der Gruppen 8 bis 12 des Periodensystems der Elemente, neben Pd in den unten angegebenen Mengen bevorzugt Pt,
(b) 8,0 g bis 100 g, bevorzugt 8,0 g bis 50 g, wenigstens eines Metalls der Gruppen 4 bis 6 und 12 des Periodensystems der Elemente, neben V in den unten angegeben Mengen bevorzugt Ti, Nb, Ta, Cr, Mo, W, und
(c) 2,0 g bis 100 g, bevorzugt 2,0 g bis 20 g, wenigstens eines Metalls der Gruppen 14 und 15 des Periodensystems der Elemente, neben Pb in den unten angegeben Mengen bevorzugt Bi,
   pro Liter Schüttvolumen des keramischen Trägers,
   wobei der Katalysator mit
(d) Kalium in einem Gehalt von 0,070 Massen-% bis 0,12 Massen-%, bezogen auf die Gesamtmasse des Katalysators und berechnet als Kalium als solches,
dotiert ist, und wobei der Katalysator
(a) 8,0 g - 50 g Palladium,
(b) 8,0 g - 50 g Vanadium,
(c) 2,0 g - 10 g Blei
pro Liter Schüttvolumen des α-Aluminiumoxids,
enthält.

Ein weiterer Gegenstand der vorliegenden Erfindung ist ein Verfahren zur Herstellung von aromatischen Aminen der Formel 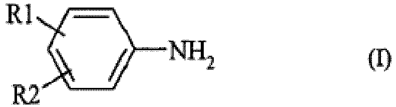 in der R1 und R2 unabhängig voneinander Wasserstoff, Methyl oder Ethyl bedeuten, wobei R1 zusätzlich NH₂ bedeuten kann, durch Hydrierung von Nitroaromaten der Formel 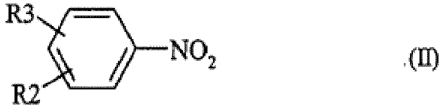 in der R2 und R3 unabhängig voneinander Wasserstoff, Methyl oder Ethyl bedeuten, wobei R3 zusätzlich NO₂ bedeuten kann,
mit Wasserstoff in Gegenwart des erfindungsgemäßen Katalysators.

Als keramisches Trägermaterial wird α-Aluminiumoxid eingesetzt.

Für die *BET-Oberfläche* ist der nach DIN ISO 9277 (Mai 2003) bestimmte Wert maßgeblich.

Das *Schüttvolumen des Trägers* wird gemäß der Formel "Schüttvolumen = Masse / Schüttdichte" berechnet aus der nach EN ISO 60 ermittelten Schüttdichte des Trägers. Die Mengenangaben für die Komponenten (a) bis (c) in g pro Liter Schüttvolumen beziehen sich auf die Metalle als solche (und nicht etwa bspw. auf ihre Oxide). Damit ist jedoch nicht gesagt, dass die Metalle notwendigerweise in elementarer Form auf dem Träger vorliegen. Falls mehrere Vertreter einer Komponente (a) bis (c) vorhanden sind, beziehen sich Mengenangaben in g pro Liter Schüttvolumen des Trägers jeweils auf die Summe aller Vertreter einer Komponente (a) bis (c), d. h. wenn bspw. Blei und Bismut als Komponente (c) vorhanden sind, liegt der Gesamtgehalt dieser beiden Komponenten im Bereich von > 2,0 g bis 100 g, bevorzugt im Bereich von > 2,0 g bis 20 g, pro Liter des Schüttvolumens des Trägers.

Die *Gruppenbezeichnungen für das Periodensystem der Elemente* richten sich im Rahmen der vorliegenden Erfindung nach der IUPAC-Empfehlung von 1986.

Das Dotierungselement *Kalium* (d) wird in Form eines Kaliumkationen enthaltenden Kaliumsalzes in den Katalysator eingeführt (siehe weiter unten für Details). Die chemische Natur des Anions des Kaliumsalzes kann sich im Laufe der Zeit, abhängig von den Einsatzbedingungen des Katalysators, ändern. Die Mengenangaben für das Dotierungselement Kalium (d) in Massen-% beziehen sich auf Kalium als solches (also nicht auf das in der Herstellung eingesetzte Kaliumsalz).

Nachstehend werden Ausführungsformen der Erfindung beschrieben. Verschiedene Ausführungsformen können dabei beliebig miteinander kombiniert werden, sofern sich aus dem Kontext nicht eindeutig das Gegenteil ergibt.

Methoden zur Herstellung erfindungsgemäßer Edelmetall-Träger-Katalysatoren sind prinzipiell bekannt. Bevorzugt wird zunächst eine Katalysator-Vorläuferverbindung umfassend den keramischen Träger und die Komponenten (a) bis (c) so hergestellt wie in DE 28 49 002 A1, insbesondere auf S. 12, Z. 1 bis S. 13 Z. 16, beschrieben, wobei der Katalysator nicht notwendigerweise erst im Reaktor getrocknet werden muss. Die Vorbehandlung des Trägermaterials mit einer Base ist dabei zwar bevorzugt, aber nicht unbedingt erforderlich. Es sich als vorteilhaft erwiesen, wenn die aktiven Komponenten des Katalysators möglichst nahe an der Trägerformkörperoberfläche in einer schmalen Zone niedergeschlagen vorliegen und das Innere des Trägermaterials kein Metall enthält.

Die Metalle können einzeln oder als Mischung in Form von Lösungen ihrer Salze auf den Träger aufgebracht werden. Als Salze sind beispielsweise die Halogenide, Acetate, Carbonate, Hydrogencarbonate, Sulfate, Phosphate, Oxalate, Formiate, Oxide und Hydroxide geeignet. Nach jedem Tränkgang und/oder zum Abschluss wird eine Reduktion durchgeführt, zu der Wasserstoff, Hydrazin und/oder Ameisensäure eingesetzt wird.

Prinzipiell können die erfindungsgemäßen Trägerkatalysatoren jede beliebige Form aufweisen, wie Kugeln, Stäbchen, Raschigringe, Granulat oder Tabletten. Bevorzugt werden Formkörper benutzt, deren Schüttungen einen niedrigen Strömungswiderstand bei gutem Gas-Oberflächen-Kontakt aufweisen, wie Raschigringe, Sattelkörper, Wagenräder und/oder Spiralen. Besonders bevorzugt werden Katalysatorformkörper verwendet, die im Wesentlichen kugelförmig sind und eine durch Siebanalyse (DIN 66 165 vom April 1987) bestimmte massenbezogene mittlere Partikelgröße x_{50,3} zwischen 1,0 mm und 10 mm haben. Die erfindungsgemäßen Katalysatoren können rein oder in Verdünnung mit anderen inerten Füllkörpern z. B. aus Glas, Keramik oder Metall eingesetzt werden.

Nach Fertigung der Katalysatorvorläuferverbindung umfassend die Komponenten (a) bis (c) auf einem keramischen Träger wird die Dotierung mit Kalium durchgeführt. Hierzu wird die Katalysatorvorläuferverbindung mit einer wässrigen Lösung eines Kaliumsalzes so getränkt, dass die Lösung vollständig aufgesaugt wird. Ggf. ist das Saugvermögen der Katalysatorvorläuferverbindung in Vorversuchen zu ermitteln (im Allgemeinen entspricht dieses dem Saugvermögen des Trägers, sodass dieser für die Vorversuche eingesetzt werden kann). Im Anschluss wird der feuchte Katalysator bis zur Gewichtskonstanz getrocknet. Dies kann in einem Ofen bei Temperaturen zwischen 50 °C und 200 °C, bevorzugt zwischen 100 °C und 150 °C und/oder in einem Strom warmer Luft oder eines warmen Inertgases geschehen.

Bevorzugte Kaliumsalze für die Dotierung sind solche, die einen hohen Kaliumgehalt haben (bezogen auf die Formelmasse), die kommerziell verfügbar, leicht in Wasser löslich und wenig oder gar nicht toxisch sind, insbesondere Kaliumsulfat, Kaliumchlorid, Kaliumhydroxid, Kaliumcarbonat, Kaliumhydrogencarbonat, Kaliumbromid, Kaliumacetat, Kaliumformiat und/oder Kaliumnitrat. Bevorzugt sind Katalysatoren, bei denen das Dotierungselement Kalium d) in Form von Kaliumsulfat oder Kaliumchlorid eingeführt wurde.

Erfindungsgemäß umfasst ein Katalysator α-Aluminiumoxid mit einer BET-Oberfläche von weniger als 10 m²/g als Träger, und
(a) 8,0 g- 50 g Palladium,
(b) 8,0 g - 50 g Vanadium,
(c) 2,0 g-10 g Blei
   pro Liter Schüttvolumen des α-Aluminiumoxids,
   wobei der Katalysator mit
(d) Kalium in einem Gehalt von 0,070 Massen-% bis 0,12 Massen-%, bezogen auf die Gesamtmasse des Katalysators,
dotiert ist.

Die zuvor beschriebenen erfindungsgemäßen Katalysatoren eignen sich als Katalysatoren für kontinuierliche Gasphasenhydrierungen von Nitroverbindungen der allgemeinen Formel II 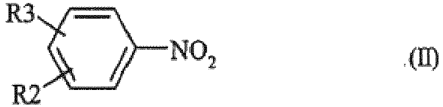 in der R2 und R3 unabhängig voneinander Wasserstoff, Methyl oder Ethyl bedeuten, wobei R3 zusätzlich NO₂ bedeuten kann. Nicht umgesetzter Wasserstoff (sowie ggf. zugesetzte Verdünnungsgase, siehe unten) kann dabei in bestimmten Ausführungsformen, ggf. nach Ausschleusung eines kleinen Teilstroms zwecks Vermeidung der Akkumulation unerwünschter Bestandteile, in die Reaktion zurückgeführt werden (Kreisgas-Fahrweise). Bevorzugt wird im Rahmen der vorliegenden Erfindung Anilin durch Hydrierung von Nitrobenzol hergestellt. Es zeigt sich dabei, dass erheblich weniger Phenol als Nebenprodukt entsteht, ohne dass die Betriebszeit des Katalysators signifikant beeinträchtigt würde. Da sich Phenol besonders schwierig durch Destillation von Anilin abtrennen lässt, ist dieser Effekt vorteilhaft, selbst wenn die Bildung anderer phenolischer Verbindungen wie Aminophenol nicht beeinflusst wird oder sogar leicht zunimmt.

Die Gasphasenhydrierung erfolgt dabei bevorzugt nach einem Verfahren, bei dem der Katalysator in einer stationären Schüttung angeordnet ist. Die Verwendung von beweglichen Katalysatoren etwa in Wirbelschicht- oder Wanderbett-Verfahren ist jedoch ebenfalls möglich. Die Verfahrensführung erfolgt entweder isotherm (d. h. unter weitest möglicher Abführung der entstehenden Reaktionswärme durch ein Kühlmedium, bspw. durch ein Wärmeträgeröl in einem Rohrbündelreaktor) oder adiabat (d. h. in isolierten Reaktoren ohne Vorrichtungen zur Wärmeabfuhr, bei denen die Reaktionswärme mit den Reaktionsgasen ausgetragen wird). Das isotherme Verfahren ist bevorzugt. Beispiele für isotherme Verfahren, in denen der erfindungsgemäße Katalysator eingesetzt werden kann, finden sich unter anderem in EP 0 011 090 A1, DE 19 715 746 A1 und EP 0 944 578 B1. Adiabate Verfahren, in denen der erfindungsgemäße Katalysator eingesetzt werden kann, sind in EP 0 696 573 B1**,** EP 0 696 574 B1 und insbesondere in EP 1 882 681 A1 beschrieben.

Die Verdampfung des Nitroaromaten kann nach dem Stand der Technik in bekannten Verdampfern erfolgen, wie z. B. Fallfilm-, Steigrohr-, Einspritz-, Dünnschicht-, Umlauf- und Wendelrohrverdampfern. Der Verdampfung kann eine grundsätzlich bekannte Tröpfchenabscheidung nachgeschaltet werden. Bei Kreisgas-Fahrweise kann die Eindosierung des Nitroaromaten so erfolgen, wie in DE-OS-1 809 711 beschrieben, bevorzugt wird jedoch der Nitroaromat im Frischwasserstoff vollständig verdampft und dann gasförmig in den Kreisgasstrom gegeben. Der Vorteil dieser Verfahrensweise liegt in der deutlich geringeren Bildung von Ablagerungen im Reaktor und in den Zuleitungen. Der Eduktgasstrom wird in bekannter Weise mittels entsprechender Zuführung und Verteilung und/oder durch Vermischungseinrichtungen im Kreisstrom vermischt.

Weiterhin ist die Verdüsung des flüssigen Nitroaromaten in den Frischwasserstoff- oder Kreisgaswasserstoffstrom mittels Einstoff- oder Zweistoffdüsen möglich, wobei die Vereinigung des Eduktgasstromes nach einer Überhitzung in einem Wärmeaustauscher erfolgen kann.

Das einzusetzende molare Verhältnis von Wasserstoff zu Nitrogruppen ist abhängig von der Verfahrensweise. Im isothermen Verfahren beträgt das molare Verhältnis von Wasserstoff zu Nitrogruppen erfindungsgemäß 3 : 1 bis 30 : 1, bevorzugt 4 : 1 bis 20 : 1, besonders bevorzugt 5 : 1 bis 10 : 1. Im adiabaten Verfahren beträgt das molare Verhältnis von Wasserstoff zu Nitrogruppen erfindungsgemäß 3 : 1 bis 150 : 1, bevorzugt 6 : 1 bis 125 : 1, ganz besonders bevorzugt 12 : 1 bis 100 : 1 und außerordentlich ganz besonders bevorzugt 50 : 1 bis 90 : 1.

Hierbei kann die Wasserstoffkonzentration durch Zumischen von Inertgasen, wie Stickstoff, Helium, Argon und/oder Wasserdampf, herabgesetzt werden. Bevorzugt wird Stickstoff als Inertgas zugemischt. Pro Mol Wasserstoff können bis zu 10 Mol, bevorzugt bis zu 3 Mol, besonders bevorzugt bis zu 1 Mol, inertes Verdünnungsgas zugemischt werden. In adiabaten Verfahren ist darauf zu achten, dass die Reaktionswärme mit dem Reaktionsgasen ausgetragen werden muss, d. h. wenn niedrige molare Verhältnisse von Wasserstoff zu Nitrogruppen eingesetzt werden sollen, müssen die genannten Verdünnungsgase in ausreichender Menge zugesetzt werden, um den adiabaten Temperatursprung in solchen Grenzen zu halten, dass die maximale Katalysator-Temperatur (siehe unten) nicht überschritten wird.

Als Reaktoren für das erfindungsgemäße Verfahren können bei isothermer Betriebsweise alle bekannten Reaktoren eingesetzt werden, die für Gasphasenreaktionen mit gekühlten stationären Katalysatorschüttungen geeignet sind. Geeignet sind z. B. Rohrbündelreaktoren, in denen sich der Katalysator innerhalb von Wärmeträger umspülten Rohren befindet und Reaktoren, in denen umgekehrt der Wärmeträger innerhalb der Rohre fließt und der Katalysator sich außerhalb der Rohre befindet. Beispielsweise sind solche Reaktoren aus DE 28 48 014 A1 und DE 30 07 202 A1 bekannt. Die Länge der Katalysatorschüttung in Strömungsrichtung liegt beim erfindungsgemäßen Verfahren bei 0,5 m bis 20 m, bevorzugt 1 m bis 10 m, besonders bevorzugt bei 2 m bis 6 m. Die Schüttungslänge kann ggf. auch durch mehrere hintereinander geschaltete Reaktoren erreicht werden.

Als Reaktoren für das erfindungsgemäße Verfahren können bei adiabater Betriebsweise einfache Reaktoren, in denen der Katalysator in Form einer Schüttung zwischen einfachen Auflagerosten und/oder Metallsieben angeordnet ist, verwendet werden. Ein Wärmeträgerkreislauf innerhalb des Reaktors entfällt völlig, da sich die Reaktionsenthalpie - ggf. bis auf geringfügige, unvermeidbare Wärmeverluste - quantitativ in der Temperaturdifferenz zwischen Edukt- und Produktgasstrom widerspiegelt. Reaktoren solchen Typs sind in allen Größen preiswert und robust.

Das erfindungsgemäße Verfahren wird bei maximalen Katalysator-Temperaturen von 600 °C, bevorzugt 550 °C, besonders bevorzugt 500 °C und außerordentlich besonders bevorzugt 460 °C betrieben. Dies gilt für die isotherme und adiabate Verfahrensweise gleichermaßen. Die maximale Katalysator-temperatur bezieht sich im isothermen Verfahren auf kurzzeitige Temperaturspitzen (sog. Hotspots) im Katalysatorbett, die sich großtechnisch auch mit optimierten Kühlkreisläufen nicht immer vermeiden lassen. Solche Temperaturspitzen werden jedoch rasch wieder abgekühlt, sodass im isothermen Verfahren die Austrittstemperatur des Produktgasgemisches im Wesentlichen gleich der Eingangstemperatur des Eduktgasgemisches ist. Die Eingangstemperatur des gasförmigen Reaktionsgemisches liegt zwischen 200 °C und 460 °C, bevorzugt zwischen 210 °C und 440 °C, besonders bevorzugt zwischen 215 °C und 300 °C und außerordentlich besonders bevorzugt zwischen 220 °C und 260 °C. Dies gilt für die isotherme und adiabate Verfahrensweise gleichermaßen. In isothermen Verfahren kann es von Vorteil sein, wenn die Temperatur des Kühlmediums während einer Fahrperiode (siehe unten) kontinuierlich oder schrittweise angehoben wird.

Bei der bevorzugten Verwendung stationärer Katalysatorschüttungen ist der eingesetzte Katalysator nach einer bestimmten Zeitspanne *(Betriebszeit)* so desaktiviert, dass kein zufriedenstellender Umsatz an Nitroverbindung mehr erzielt wird. Dann wird die Reaktion unterbrochen und der Katalysator regeneriert. Nach erfolgter Regeneration kann die Reaktion in einer neuen *Fahrperiode* wieder angefahren werden.

Die Regenerierung desaktivierter Katalysatorschüttungen erfolgt mit Stickstoff/Luftmischungen bei Temperaturen von 200 °C bis 400 °C, bevorzugt bei 250 °C bis 350 °C, ohne Ausbau des Katalysators aus dem Reaktor. Dabei wird im Allgemeinen bei Stickstoff-Gehalten von 85 Vol.-% bis 100 Vol.-% im Gasstrom begonnen und der Sauerstoff-Gehalt während des Abbrennens schrittweise auf den Gehalt reiner Luft angehoben. Gegebenenfalls können am Ende der Regenerierung mit reinem Sauerstoff hartnäckige Verkokungen abgebrannt werden. Anstelle von Stickstoff können auch andere inerte Gase zu Sauerstoff oder Luft zugemischt werden, wie Argon, Helium und/oder Wasserdampf.

Die in bestimmten Ausführungsformen übliche Verdünnung des Reaktionsgemisches mit einem inerten Gas wird bevorzugt zu Beginn einer Fahrperiode mit frischem Katalysator und nach dem Regenerieren des Katalysators durch Abbrennen mit Luft und Reduzierung mit Wasserstoff angewendet. Bevorzugt wird in den ersten 300 Stunden, besonders bevorzugt in den ersten 200 Stunden, insbesondere in den ersten 100 Stunden, nach dem Wiederanfahren mit einem Inertgas verdünnt.

Bei der bevorzugten isothermen Fahrweise liegt der absolute Gesamtdruck bei Eintritt in den Reaktor zwischen 0,500 bar und 6,00 bar, bevorzugt zwischen 1,10 bar und 3,00 bar, besonders bevorzugt zwischen 1,20 bar und 2,00 bar. Bei der adiabaten Fahrweise liegt der absolute Gesamtdruck bei Eintritt in den Reaktor zwischen 1,00 bar und 50,0 bar, bevorzugt zwischen 2,00 bar und 20,0 bar, besonders bevorzugt zwischen 2,00 bar und 10,0 bar.

Die Belastung der erfindungsgemäßen Katalysatoren mit eingesetzter aromatischer Nitroverbindung sollte kontinuierlich oder stufenweise von 0,010 kg_{Nitroverbindung}/(l_{Katalysator} · h) bis 0,20 kg_{Nitroverbindung}/(l_{Katalysator} · h) auf 0,50 kg_{Nitroverbindung}/(l_{Katalysator} · h) bis 5,0 kg_{Nitroverbindung}/(l_{Katalysator} · h) erhöht werden, wobei die maximale Belastung innerhalb von 10 bis 1 000 Stunden erreicht wird. Die Angabe "l_{Katalysator}" bezieht sich dabei auf das Schüttvolumen des Katalysators, welches in diesem Fall identisch ist mit dem Innenvolumen des Teils des Reaktors, der mit Katalysator gefüllt ist.

Die hohe Endbelastung wird bis zum Durchbruch nicht umgesetzter Nitroverbindung konstant gehalten. Wenn die Eduktkonzentration am Reaktorende einen zu hohen Wert annimmt kann bei isothermer Fahrweise die Temperatur des Wärmeträgers angehoben werden. Alternativ oder zusätzlich kann auch die Belastung an Nitroverbindung gesenkt werden, um eine Produktionsunterbrechung zur Regeneration des Katalysators hinauszuzögern. Die letztgenannte Möglichkeit lässt sich natürlich auch bei adiabater Fahrweise umsetzen. Weitere Möglichkeiten bei adiabater Fahrweise umfassen das Anheben der Eintrittstemperatur und das Zulassen eines höheren Temperatursprungs. Wann ein "zu hoher" Wert an Nitroaromat im Produktstrom erreicht ist, hängt von den konkreten Bedingungen einer Produktionsanlage ab, bspw. der Leistungsfähigkeit der nachgeschalteten Destillation oder auch dem angestrebten Einsatzgebiet des aromatischen Amins. Unter Umständen kann es sinnvoll sein, bereits bei den ersten Anzeichen von nicht umgesetzten Nitroaromaten im Produkt (d. h. in der Größenordnung von 10 ppm Nitroaromat) eine oder mehrere der geschilderten Maßnahmen durchzuführen.

Die technische Verwirklichung eines Verfahrens mit den erfindungsgemäßen Katalysatoren kann beispielsweise wie folgt erfolgen: Ein Kreisgasstrom, im Wesentlichen bestehend aus Wasserstoff und wenig Wasser, wird verdichtet, um die Strömungswiderstände der Anlage zu überwinden. Mittels Gegenstromwärmetauschung wird der Gasstrom aufgeheizt, wobei die Wärme z. B. dem Kreisgasstrom vor der Kondensation der Produkte entnommen wird. Der Kreisgasstrom wird auf die gewünschte Temperatur gebracht. Im Frischwasserstoff, der den verbrauchten ersetzt, wird der zu hydrierende Nitroaromat verdampft, überhitzt und anschließend mit dem Kreisgasstrom vermischt. Das Gasgemisch wird in einen thermostatisierten Reaktor (z. B. Rohrbündelreaktor) mit stationär angeordnetem Katalysator (isotherme Fahrweise) oder in einen gut isolierten Reaktor ohne Vorrichtung zur Wärmeabfuhr (adiabate Fahrweise) eingeleitet. Die freiwerdende Reaktionswärme wird bei isothermer Fahrweise mittels eines Wärmeträgers (z. B. Wärmeträgeröl oder Salzschmelze) dem Reaktor entnommen; bei adiabater Fahrweise spiegelt sie sich in einem entsprechenden Temperaturanstieg des Produktgasstroms (adiabater Temperatursprung) wider. Der den Reaktor verlassende Produktstrom wird zum Aufheizen des Kreisgasstroms genutzt und dann bis zur Kondensation von gebildeten Amin und Wasser abgekühlt. Die Flüssigkeiten werden ausgeschleust, ebenso eine kleine Menge Kreisgas zum Entfernen von inerten Gasen, z. B. Stickstoff oder Ammoniak, die sich sonst anreichern würden. Das Kreisgas wird anschließend wieder dem Verdichter zugeführt.

Das erfindungsgemäße Verfahren mit den neuen Katalysatoren zeichnet sich insbesondere durch einen geringen Gehalt an phenolischen Nebenprodukten (insbesondere einem geringerem Gehalt an Phenol) in den Rohprodukten aus, wie durch die Beispiele veranschaulicht wird.

### Beispiele

### Beispiel 1: Katalysatorpräparation

### la) Ohne Kalium, gemäß DE 28 49 002 A1 (Vergleich)

1,00 Liter (Schüttvolumen) eines α-Al₂O₃-Trägers in Kugelform mit 3,0 bis 5,0 mm Durchmesser (x_{50,3}), einer BET-Oberfläche von 9,8 m²/g, einer Saugfähigkeit von 45,1 ml Wasser pro 100 g Träger und einer Schüttdichte von 812 g/l wurde mit 366 ml einer 10,8 g (entsprechend 0,27 mol) NaOH enthaltenden wässrigen Lösung imprägniert. Die Lösung wurde innerhalb weniger Minuten völlig vom Träger aufgenommen.

Der feuchte Träger wurde in einem warmen aufsteigenden starken Luftstrom getrocknet. Die Trockenzeit bis zur Gewichtskonstanz betrug ungefähr 15 Minuten. Der Restfeuchtegehalt lag nach dem Abkühlen etwa bei 1 % der Saugfähigkeit des Trägers.

Der so vorbehandelte trockene Träger wurde seiner Saugfähigkeit entsprechend mit 366 ml einer wässrigen Natriumtetrachloropalladat-Lösung, die 9,0g Palladium (entsprechend 0,846 mol) enthielt, getränkt und 15 Minuten stehen gelassen. Zur Reduktion der auf dem Träger abgeschiedenen Palladiumverbindung zu metallischem Palladium wurde der Katalysator mit 400 ml einer 10%igen wässrigen Hydrazinhydrat- Lösung überschichtet und 2 Stunden stehen gelassen. Danach wurde der Katalysator mit vollentsalztem Wasser gründlich gespült, bis keine Ionen der bei der Katalysatorherstellung verwendeten Verbindungen im Waschwasser mehr nachweisbar waren was nach ca. 10 Stunden der Fall war.

Anschließend wurde wieder in einem starken warmen aufsteigenden Luftstrom bis zur Gewichtskonstanz getrocknet.

Der Pd-haltige Katalysator wurde anschließend mit 366 ml einer wässrigen, 9,0 g Vanadium als Vanadyloxalat enthaltenden Lösung getränkt. Die Trocknung des Trägers im Warmluftstrom erfolgte wie oben angegeben. Anschließend wurde der Katalysator in einem Rohrofen bei 300 °C 6 Stunden thermisch behandelt, wobei das Oxalat zersetzt wurde.

Abschließend wurde der Katalysator mit 366 ml einer wässrigen, 3,0 g Blei als Bleiacetat enthaltenden Lösung getränkt und wiederum im aufsteigenden Luftstrom getrocknet.

Der fertige Katalysator enthielt 9,0 g Pd, 9,0 g Vanadium und 3,0 g Blei pro Liter Schüttvolumen des Trägers und entsprach dem Katalysator aus Beispiel 1 in DE 28 49 002 A1.

### 1b) 0,090 Massen-% Kalium als Kaliumsulfat (erfindungsgemäß)

1,000 kg eines Katalysators gemäß Beispiel 1 wurde mit einer Lösung aus 2,0 g K₂SO₄ in 400 ml Wasser getränkt. Anschließend wurde der Katalysator im Warmluftstrom bis zur Gewichtskonstanz getrocknet. Der Katalysator enthielt 0,037 Massen-% Schwefel.

### lc) 0,0090 Massen-% Kalium als Kaliumsulfat (Vergleich)

1,000 kg eines Katalysators gemäß Beispiel 1 wurde mit einer Lösung aus 0,20 g K₂SO₄ in 400 ml Wasser getränkt. Anschließend wurde der Katalysator im Warmluftstrom bis zur Gewichtskonstanz getrocknet. Der Katalysator enthielt 0,0037 Massen-% Schwefel.

### 1d) 0,44 Massen-% Kalium als Kaliumsulfat (Vergleich)

1,000 kg eines Katalysators gemäß Beispiel 1 wurde mit einer Lösung aus 10,0 g K₂SO₄ in 400 ml Wasser getränkt. Anschließend wurde der Katalysator im Warmluftstrom bis zur Gewichtskonstanz getrocknet. Der Katalysator enthielt 0,18 Massen-% Schwefel und liegt damit im Vorzugsbereich des in EP 1 882 681 A1 offenbarten Schwefelgehalts von 0,1 bis 1 Massen-%.

### le) Dotierung mit Natrium als Natriumsulfat (Vergleich)

1,000 kg eines Katalysators gemäß Beispiel 1 wurde mit einer Lösung aus 2,0 g Na₂SO₄ in 400 ml Wasser getränkt. Anschließend wurde der Katalysator im Warmluftstrom bis zur Gewichtskonstanz getrocknet. Der Katalysator enthielt 0,045 Massen-% Schwefel.

### 1f) 0,10 Massen-% Kalium als Kaliumchlorid (erfindungsgemäß)

1,000 kg eines Katalysators gemäß Beispiel 1 wurde mit einer Lösung aus 2,00 g KCl in 400 ml Wasser getränkt. Anschließend wurde der Katalysator im Warmluftstrom bis zur Gewichtskonstanz getrocknet.

### Beispiel 2: Nitrobenzolhydrierung

### Allgemeine Versuchsbedingungen

Als Reaktor diente ein mit Öl thermostatisiertes Rohr mit einem Innendurchmesser von ca. 26 mm, in das eine 285 cm hohe Schüttung des jeweiligen Katalysators eingebracht wurde. Der Katalysator wurde vor jedem Versuch zunächst mit Stickstoff, dann mit Wasserstoff gespült und dann im Wasserstoffstrom von ca. 1500 NL/h (Normliter pro Stunde) in 5 Stunden auf 240 °C aufgeheizt. Anschließend wurde begonnen, Nitrobenzol im Wasserstoffstrom zu verdampfen. Die Nitrobenzol-Wasserstoff-Mischung gelangte mit ca. 230 °C auf die Oberfläche der Katalysatorschüttung. Die spezifische Belastung des Katalysators wurde zu Beginn jedes Versuchs schrittweise von 0,20 kg_{Nitrobenzol}/(l_{Katalysator} · h) auf 1,0 kg_{Nitrobenzol}/(l_{Katalysator} · h) erhöht. Dies erfolgte in der Art, dass der Katalysator an keiner Stelle wärmer als 400 °C wurde. Die Änderung der Öltemperatur entlang des Reaktorrohres betrug ca. ± 1 K. Die Strömungsgeschwindigkeit des Öls entlang der Rohroberfläche betrug ca. 1,5 m/s.

Gegebenenfalls durchgeführte Regenerierungen des Katalysators erfolgten nach Inertisierung des Reaktors mit Stickstoff. Der Reaktor wurde im Stickstoffstrom von 1500 NL/h auf 270 °C temperiert. Dann wurde der Stickstoffstrom innerhalb von 12 h schrittweise auf 0 NL/h reduziert und im gleichen Zeitraum ein Luftstrom von 0 NL/h schrittweise auf 500 NL/h erhöht. Der Reaktor wurde weitere 24 h lang bei 270 °C mit 500 NL/h Luft beaufschlagt, um Verkokungen abzubrennen.

Umsätze, Selektivitäten und Phenolgehalte wurden mittels Gaschromatographie bestimmt.

### 2a) Katalysator aus Beispiel la (Vergleich)

### 2aa) Erste Fahrperiode

Der Versuch wurde entsprechend den allgemeinen Versuchsbedingungen durchgeführt. Nach 750 h wurde begonnen, die Öltemperatur in Schritten von 5 K pro Tag zu erhöhen, bis 300 °C Öltemperatur erreicht wurden. Der Katalysator erreichte eine Betriebszeit von ca. 1080 Stunden, nach denen der Nitrobenzol-Gehalt des Kondensates von 0 auf ca. 300 ppm anstieg und der Katalysator daraufhin durch Abbrennen regeneriert werden musste. Die durchschnittliche Selektivität betrug 99,63 %, und der durchschnittliche Phenolgehalt im Rohanilin lag bei 194 ppm.

### 2ab) Zweite Fahrperiode

In der zweiten Fahrperiode, also nach dem Regenerieren, steigerte sich die Betriebszeit auf ca. 1340 h. Die Erhöhung Öltemperatur erfolgte in Schritten von 5 K pro Tag, beginnend nach 1000 h bis 300 °C Öltemperatur erreicht waren. Die durchschnittliche Selektivität betrug 99,61 %, und der durchschnittliche Phenolgehalt im Rohanilin lag bei 159 ppm.

### 2b) Katalysator aus Beispiel 1b (erfindungsgemäß)

### 2ba) Erste Fahrperiode

Der Versuch wurde entsprechend den allgemeinen Versuchsbedingungen durchgeführt. Nach 750 h wurde begonnen, die Öltemperatur in Schritten von 5 K pro Tag zu erhöhen, bis 300 °C Öltemperatur erreicht wurden. Der Katalysator erreichte eine Betriebszeit von ca. 1090 Stunden, nach denen der Nitrobenzol-Gehalt des Kondensates von 0 auf ca. 300 ppm anstieg und der Katalysator daraufhin durch Abbrennen regeneriert werden musste. Die durchschnittliche Selektivität betrug 99,72 %, und der durchschnittliche Phenolgehalt im Rohanilin lag bei 109 ppm.

### 2bb) Zweite Fahrperiode

In der zweiten Fahrperiode, also nach dem Regenerieren, steigerte sich die Betriebszeit auf ca. 1320 h. Die Erhöhung Öltemperatur erfolgte in Schritten von 5 K pro Tag, beginnend nach 1050 h bis 300 °C Öltemperatur erreicht waren. Die durchschnittliche Selektivität betrug 99,74 %, und der durchschnittliche Phenolgehalt im Rohanilin lag bei 74 ppm.

### 2bc) Dritte Fahrperiode

In der dritten Fahrperiode, also nach dem zweiten Regenerieren, steigerte sich die Betriebszeit auf ca. 1420 h. Die Erhöhung Öltemperatur erfolgte in Schritten von 5 K pro Tag, beginnend nach 1150 h bis 300 °C Öltemperatur erreicht waren. die durchschnittliche Selektivität betrug 99,79 %, und der durchschnittliche Phenolgehalt im Rohanilin 50 ppm.

### 2c) Katalysator aus Beispiel 1c (Vergleich)

Der Versuch wurde entsprechend den allgemeinen Versuchsbedingungen durchgeführt. Nach 725 h wurde begonnen, die Öltemperatur in Schritten von 5 K pro Tag zu erhöhen, bis 300 °C Öltemperatur erreicht wurden. Der Katalysator erreichte eine Betriebszeit von ca. 1100 Stunden, nach denen der Nitrobenzol-Gehalt des Kondensates von 0 auf ca. 300 ppm anstieg und der Katalysator daraufhin durch Abbrennen regeneriert werden musste. Die durchschnittliche Selektivität betrug 99,70 %, und der durchschnittliche Phenolgehalt im Rohanilin 147 ppm.

### 2d) Katalysator aus Beispiel 1d (Vergleich)

Der Versuch wurde entsprechend den allgemeinen Versuchsbedingungen durchgeführt. Nach 580 h wurde begonnen, die Öltemperatur in Schritten von 5 K pro Tag zu erhöhen, bis 300 °C Öltemperatur erreicht wurden. Der Katalysator erreichte eine Betriebszeit von ca. 870 Stunden, nach denen der Nitrobenzol-Gehalt des Kondensates von 0 auf ca. 300 ppm anstieg und der Katalysator daraufhin durch Abbrennen regeneriert werden musste. Die durchschnittliche Selektivität betrug 99,92 %, und der durchschnittliche Phenolgehalt im Rohanilin lag bei 22 ppm.

### 2e) Katalysator aus Beispiel 1e (Vergleich)

Der Versuch wurde entsprechend den allgemeinen Versuchsbedingungen durchgeführt. Nach 530 h wurde begonnen, die Öltemperatur in Schritten von 5 K pro Tag zu erhöhen, bis 300 °C Öltemperatur erreicht wurden. Der Katalysator erreichte eine Betriebszeit von ca. 870 Stunden, nach denen der Nitrobenzol-Gehalt des Kondensates von 0 auf ca. 300 ppm anstieg und der Katalysator daraufhin durch Abbrennen regeneriert werden musste. Die durchschnittliche Selektivität betrug 99,75 %, und der durchschnittliche Phenolgehalt im Rohanilin lag bei 98 ppm.

### 2f) Katalysator aus Beispiel 1f (erfindungsgemäß)

Der Versuch wurde entsprechend den allgemeinen Versuchsbedingungen durchgeführt. Nach 530 h wurde begonnen, die Öltemperatur in Schritten von 5 K pro Tag zu erhöhen, bis 300 °C Öltemperatur erreicht wurden. Der Katalysator erreichte eine Betriebszeit von ca. 1150 Stunden, nach denen der Nitrobenzol-Gehalt des Kondensates von 0 auf ca. 300 ppm anstieg und der Katalysator daraufhin durch Abbrennen regeneriert werden musste. Die durchschnittliche Selektivität betrug 99,86 %, und der durchschnittliche Phenolgehalt im Rohanilin lag bei 102 ppm.

Die folgende Tabelle stellt die Resultate einander gegenüber.

**Tabelle:**

| **Bei spiel** | **Katalysator** | **Dotierungsmittel** | **Betriebszeit (Umsatz > 99,97 %)** | **Durch schnittliche Selektivität** | **Durch- schnittlicher Phenolgehalt** |
|---|---|---|---|---|---|
| | | (g_{Dotierungsmittel}/ kg_{Katalysatorvorläuferverbindung}) | [h] | [%] | [ppm] |
| 2aa (Vgl.) | 1a | - | 1080 | 99,63 | 194 |
| 2ab (Vgl.) | 1a (regeneriert) | | 1340 | 99,61 | 159 |
| 2ba (erf.) | 1b | | 1090 | 99,72 | 109 |
| 2bb (erf.) | 1b (regeneriert) | K₂SO₄ (2,0) | 1320 | 99,74 | 74 |
| 2bc (erf.) | 1b (2x regeneriert) | | 1420 | 99,79 | 50 |
| 2c (Vgl.) | 1c | K₂SO₄ (0,20) | 1100 | 99,70 | 147 |
| 2d (Vgl.) | 1d | K₂SO₄ (10,0) | 870 | 99,92 | 22 |
| 2e (Vgl.) | 1e | Na₂SO₄ (2,0) | 830 | 99,75 | 98 |
| 2f (erf.) | 1f | KCl (2,0) | 1150 | 99,86 | 102 |

| | | | | | |
|---|---|---|---|---|---|
| (erf. = erfindungsgemäß; Vgl. = Vergleichsversuch) | | | | | |

Wie ein Vergleich von Katalysator 1a mit 1b zeigt, lässt sich durch Einsatz des mit Kalium dotierten Katalysators 1b der Phenolgehalt signifikant reduzieren, ohne dass sich die Betriebszeit nachteilig verändert (die Betriebszeiten von 2aa und 2ba sowie von 2ab und 2bb sind im Rahmen der Messgenauigkeit gleich). Eine Reduzierung des Kaliumgehalts um den Faktor 10 führt wieder zu erheblich erhöhten Phenolgehalten (vgl. 2c mit 2ba). Bei sehr hohen Kaliumgehalten sinkt der Phenolgehalt zwar drastisch (siehe 2d), jedoch um den Preis einer signifikant reduzierten Betriebszeit, was den Vorteil der verringerten Phenolbildung überkompensiert. Der Einsatz von Natriumsulfat verringert zwar ebenfalls die Phenolwerte, jedoch um den Preis einer verkürzten Betriebszeit (vgl. 2e und 2ba). Versuch 2f zeigt, dass die Effekte nicht auf Schwefel zurückzuführen sind.

## Patentansprüche

1. Katalysator umfassend α-Aluminiumoxid mit einer BET-Oberfläche von weniger als 10 m²/g als keramischen Träger und, bezogen jeweils auf die Summe aller Vertreter einer Komponente (a)-(c),
(a) 8,0 g bis 100 g wenigstens eines Metalls der Gruppen 8 bis 12 des Periodensystems der Elemente,
(b) 8,0 g bis 100 g wenigstens eines Metalls der Gruppen 4 bis 6 und 12 des Periodensystems der Elemente und
(c) 2,0 g bis 100 g wenigstens eines Metalls der Gruppen 14 und 15 des Periodensystems der Elemente,
pro Liter Schüttvolumen des keramischen Trägers,
wobei der Katalysator mit
(d) Kalium in einem Gehalt von 0,070 Massen-% bis 0,12 Massen-%, bezogen auf die Gesamtmasse des Katalysators und berechnet als Kalium als solches,
dotiert ist,
und wobei der Katalysator
(a) 8,0 g - 50 g Palladium,
(b) 8,0 g - 50 g Vanadium,
(c) 2,0 g - 10 g Blei
pro Liter Schüttvolumen des α-Aluminiumoxids,
enthält.

2. Katalysator nach Anspruch 1, bei dem der Katalysator mit Kalium (d) in Form von Kaliumsulfat, Kaliumchlorid, Kaliumhydroxid, Kaliumcarbonat, Kaliumhydrogencarbonat, Kaliumbromid, Kaliumacetat, Kaliumformiat und/oder Kaliumnitrat dotiert ist.

3. Katalysator nach Anspruch 1, bei dem der Katalysator mit Kalium (d) in Form von Kaliumsulfat oder Kaliumchlorid dotiert ist.

4. Verfahren zur Herstellung von aromatischen Aminen der Formel 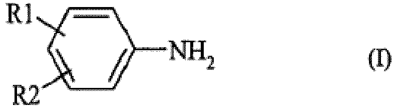 in der R1 und R2 unabhängig voneinander Wasserstoff, Methyl oder Ethyl bedeuten, wobei R1 zusätzlich NH₂ bedeuten kann, durch Hydrierung von Nitroaromaten der Formel 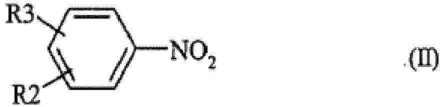 in der R2 und R3 unabhängig voneinander Wasserstoff, Methyl oder Ethyl bedeuten, wobei R3 zusätzlich NO₂ bedeuten kann,
mit Wasserstoff in Gegenwart eines Katalysators nach einem der Ansprüche 1 bis 3.

5. Verfahren nach Anspruch 4, bei dem Anilin durch Hydrierung von Nitrobenzol hergestellt wird.

6. Verfahren nach Anspruch 4 oder 5, bei dem die Hydrierung isotherm in einem Reaktor mit Abführung der entstehenden Reaktionswärme durch ein Kühlmedium durchgeführt wird.

7. Verfahren nach Anspruch 6, bei dem
das molare Verhältnis von Wasserstoff zu Nitrogruppen 3 : 1 bis 30 : 1,
der absolute Druck am Eintritt des Reaktors 0,500 bar bis 6,00 bar,
die Eingangstemperatur des gasförmigen Reaktionsgemisches 200 °C bis 460 °C,
und
die maximale Katalysator-Temperaturen 600 °C
betragen.

## Claims

1. Catalyst comprising α-aluminium oxide with a BET surface area of less than 10 m²/g as ceramic support and, based in each case on the sum total of all of the representatives of any component (a) - (c),
(a) 8.0 g to 100 g of at least one metal of groups 8 to 12 of the periodic table of the elements,
(b) 8.0 g to 100 g of at least one metal of groups 4 to 6 and 12 of the periodic table of the elements and
(c) 2.0 g to 100 g of at least one metal of groups 14 and 15 of the periodic table of the elements,
per litre of bulk volume of the ceramic support,
wherein the catalyst is doped with
(d) potassium in a content of from 0.070 % by weight to 0.12 % by weight, based on the total weight of the catalyst and calculated as potassium as such,
and wherein the catalyst contains
(a) 8.0 g - 50 g of palladium,
(b) 8.0 g - 50 g of vanadium,
(c) 2.0 g - 10 g of lead
per litre of bulk volume of the α-aluminium oxide.

2. Catalyst according to claim 1, in which the catalyst is doped with potassium (d) in the form of potassium sulfate, potassium chloride, potassium hydroxide, potassium carbonate, potassium bicarbonate, potassium bromide, potassium acetate, potassium formate and/or potassium nitrate.

3. Catalyst according to claim 1, in which the catalyst is doped with potassium (d) in the form of potassium sulfate or potassium chloride.

4. Process for the preparation of aromatic amines of the formula 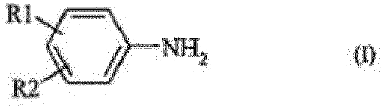 in which R1 and R2 independently of each other denote hydrogen, methyl or ethyl, wherein R1 can additionally denote NH₂, by hydrogenation of nitroaromatics of the formula 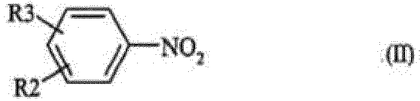 in which R2 and R3 independently of each other denote hydrogen, methyl or ethyl, wherein R3 can additionally denote NO₂,
with hydrogen in the presence of a catalyst according to one of claims 1 to 3.

5. Process according to claim 4, in which aniline is prepared by hydrogenation of nitrobenzene.

6. Process according to claim 4 or 5, in which the hydrogenation is carried out isothermally in a reactor with removal of the resulting heat of reaction by a cooling medium.

7. Process according to claim 6, in which
the molar ratio of hydrogen to nitro groups is 3 : 1 to 30 : 1,
the absolute pressure at the entry of the reactor is 0.500 bar to 6.00 bar,
the entry temperature of the gaseous reaction mixture is 200ºC to 460ºC,
and
the maximum catalyst temperatures are 600ºC.

## Revendications

1. Catalyseur comprenant de l'oxyde d'aluminium α ayant une surface BET inférieure à 10 m²/g en tant que support céramique et, à chaque fois par rapport à la somme de tous les représentants d'un composant (a) à (c),
(a) 8,0 g à 100 g d'au moins un métal des groupes 8 à 12 du tableau périodique des éléments,
(b) 8,0 g à 100 g d'au moins un métal des groupes 4 à 6 et 12 du tableau périodique des éléments, et
(c) 2,0 à 100 g d'au moins un métal des groupes 14 et 15 du tableau périodique des éléments,
par litre de volume apparent du support céramique,
le catalyseur étant dopé avec
(d) du potassium en une teneur de 0,070 % en masse à 0,12 % en masse, par rapport à la masse totale du catalyseur et calculé en tant que potassium,
et le catalyseur contenant
(a) 8,0 g à 50 g de palladium,
(b) 8,0 g à 50 g de vanadium,
(c) 2,0 g à 10 g de plomb,
par litre de volume apparent de l'oxyde d'aluminium α.

2. Catalyseur selon la revendication 1, dans lequel le catalyseur est dopé avec du potassium (d) sous la forme de sulfate de potassium, de chlorure de potassium, d'hydroxyde de potassium, de carbonate de potassium, d'hydrogénocarbonate de potassium, de bromure de potassium, d'acétate de potassium, de formiate de potassium et/ou de nitrate de potassium.

3. Catalyseur selon la revendication 1, dans lequel le catalyseur est dopé avec du potassium (d) sous la forme de sulfate de potassium ou de chlorure de potassium.

4. Procédé de fabrication d'amines aromatiques de formule 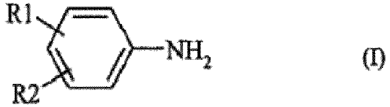 dans laquelle R1 et R2 signifient indépendamment l'un de l'autre hydrogène, méthyle ou éthyle, R1 pouvant en outre signifier NH₂, par hydrogénation de composés nitroaromatiques de formule 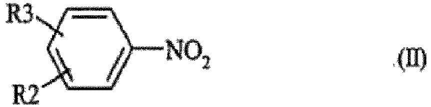 dans laquelle R2 et R3 signifient indépendamment l'un de l'autre hydrogène, méthyle ou éthyle, R3 pouvant en outre signifier NO₂,
avec de l'hydrogène en présence d'un catalyseur selon l'une quelconque des revendications 1 à 3.

5. Procédé selon la revendication 4, dans lequel de l'aniline est fabriquée par hydrogénation de nitrobenzène.

6. Procédé selon la revendication 4 ou 5, dans lequel l'hydrogénation est réalisée sous forme isotherme dans un réacteur avec dissipation de la chaleur de réaction formée par un milieu réfrigérant.

7. Procédé selon la revendication 6, dans lequel le rapport molaire entre l'hydrogène et les groupes nitro est de 3:1 à 30:1,
la pression absolue à l'entrée du réacteur est de 0,500 bar à 6,00 bar,
la température d'entrée du mélange réactionnel gazeux est de 200 °C à 460 °C,
et
les températures maximales du catalyseur sont de 600 °C.
